# EUROPEAN PATENT APPLICATION

(11) **EP 4 814 974 A1**
(43) Date of publication of application: **30.09.2026**
(21) Application number: 24924675.2
(22) Date of filing: 24.09.2024
(51) Int. Cl.: G16C 60/00

(54) **METHOD FOR ANALYZING POLYMER COMPOSITE MATERIAL IN WHICH FILLER IS DISPERSED IN MATRIX POLYMER**

(30) Priority: 16.02.2024 JP 2024021783
(71) Applicant: Sumitomo Riko Company Limited, Komaki-shi, Aichi 485-8550 (JP)
(72) Inventor: WATANABE Naoki, Komaki-shi, Aichi 485-8550 (JP); KUMAGAI Shinji, Komaki-shi, Aichi 485-8550 (JP); IWATA Yuhei, Komaki-shi, Aichi 485-8550 (JP); YAJIMA Takashi, Komaki-shi, Aichi 485-8550 (JP)
(74) Representative: Hoffmann Eitle
(86) International application number: PCT/JP2024/033797
(87) International publication number: WO 2025/173295

(57) **Abstract**

A new analysis method is provided to analyze the relationship between the dispersion form of a filler in a matrix polymer and properties of a polymer composite material. A computer-implemented method for analyzing a polymer composite material in which a filler is dispersed in a matrix polymer includes steps of: creating an analytical model of a polymer composite material in which a filler is dispersed in a matrix polymer (S1); calculating a feature representing a dispersion form of filler particle models in the analytical model (S2); performing a property simulation to calculate a physical quantity related to a mechanical property or an electrical property of the analytical model (S3); and creating a dataset including the physical quantity obtained from the property simulation and the feature representing a dispersion form of filler particle models (S4).

## Description

### TECHNICAL FIELD

The present disclosure relates to a method for analyzing a polymer composite material in which a filler is dispersed in a matrix polymer.

### BACKGROUND ART

It is known that the properties of a composite material containing a filler in a polymer vary depending on the dispersion form (morphology) of the filler. In the development of such composite materials, computer simulations, machine learning, and the like are used to analyze the effect of the dispersion forms of fillers on various properties of composite materials.

For example, JP-B-5854067 proposes a method for creating a simulation model, a simulation method, and a program that can analyze morphological contribution of heterogeneous materials to mechanical properties.

### RELATED ART DOCUMENT

### PATENT DOCUMENT

JP-B-5854067

### SUMMARY

### PROBLEMS TO BE SOLVED BY THE DISCLOSURE

However, the analysis of the dispersion forms of fillers has not been sufficiently advanced, and the design guidelines using the results of such analysis have not been established.

The present disclosure has been made in light of the aforementioned circumstances and provides a new method for analyzing the relationship between the dispersion form of a filler and various properties of a composite material containing the filler in a polymer, thereby facilitating the accumulation of knowledge on the dispersion forms and contributing to the establishment of design guidelines.

### MEANS FOR SOLVING THE PROBLEMS

The present disclosure has the following [1] to [8] as subject matters.
[1] A computer-implemented method for analyzing a polymer composite material in which a filler is dispersed in a matrix polymer,
   the method comprising the steps of:
   Creating (S1) an analytical model of a polymer composite material in which a filler is dispersed in a matrix polymer;
   Calculating (S2) a feature representing a dispersion form of filler particle models in the analytical model;
   Performing (S3) a property simulation to calculate a physical quantity related to a mechanical property or an electrical property of the analytical model; and
   Creating (S4) a dataset including the physical quantity related to a mechanical property or an electrical property obtained from the property simulation and the feature representing a dispersion form of filler particle models.
[2] The method for analyzing a polymer composite material according to [1], wherein the analytical model is an analytical model comprising a matrix polymer model and filler particle models, and
   wherein the step of creating (S4) a dataset includes creating a dataset comprising the physical quantity related to a mechanical property or an electrical property obtained from the property simulation, the feature representing a dispersion form of filler particle models, and a feature related to a number of filler particle models.
[3] The method for analyzing a polymer composite material according to [1] or [2], further comprising a step of performing (S5) multivariate analysis or machine learning using the dataset to create a predictive model.
[4] The method for analyzing a polymer composite material according to any one of [1] to [3], wherein the step of calculating (S2) a feature representing a dispersion form of filler particle models in the analytical model comprises setting vertices based on coordinates of filler particle models and calculating a feature based on Delaunay partitioning.
[5] The method for analyzing a polymer composite material according to any one of [1] to [4],
   wherein the step of calculating (S2) a feature representing a dispersion form of filler particle models in the analytical model comprises calculating a feature based on an Iδ index represented by formula (1): ${I}_{δ} = q {\sum }_{j = 1}^{q} {x}_{j} \left({x}_{j}-1\right) / {\sum }_{j = 1}^{q} {x}_{j} \left({\sum }_{j = 1}^{q} {x}_{j} - 1\right)$
   wherein in formula (1), q is a number of regions of the analytical model divided into a predetermined number of regions, and Xⱼ is a number of filler particle models in a jth region.
[6] The method for analyzing a polymer composite material according to any one of [1] to [5],
   wherein the step of calculating (S2) a feature representing a dispersion form of filler particle models in the analytical model comprises:
   calculating a feature based on at least one of a radial component and an angular component of a power spectrum obtained by a Fourier transform.
[7] The method for analyzing a polymer composite material according to any one of [1] to [6],
   Wherein the step of performing (S3) a property simulation to calculate a physical quantity related to a mechanical property of the analytical model includes
   deforming the analytical model, and
   calculating a physical quantity related to stress of the deformed analytical model.
[8] The method for analyzing a polymer composite material according to any one of [1] to [7], wherein the polymer composite material in which a filler is dispersed in a matrix polymer is a vibration isolation rubber.

### EFFECTS OF THE DISCLOSURE

The present disclosure provides a new method for analyzing the dispersion form of a filler in a polymer composite material in which the filler is dispersed in a matrix polymer, thereby facilitating the accumulation of knowledge on the dispersion forms of fillers, and contributing to the establishment of design guidelines for polymer composite materials.

According to an embodiment of the present disclosure, the prediction accuracy of a predictive model may be improved.

### BRIEF DESCRIPTION OF DRAWINGS

FIG. 1 is a flowchart illustrating an example of a processing procedure of an analysis method according to an embodiment of the present disclosure;
FIG. 2 is a diagram illustrating an example of an analytical model used in the analysis method according to an embodiment of the present disclosure;
FIG. 3 is a diagram illustrating an example of step S2a in the analysis method according to an embodiment of the present disclosure;
FIG. 4 is a diagram illustrating an example of step S2a in the analysis method according to an embodiment of the present disclosure;
FIG. 5 is a diagram illustrating an example of an analytical model used in the analysis method according to an embodiment of the present disclosure;
FIG. 6 is a diagram illustrating an example of step S2b in the analysis method according to an embodiment of the present disclosure;
FIG. 7 is a diagram illustrating an example of step S2b in the analysis method according to an embodiment of the present disclosure;
FIG. 8 is a diagram illustrating an example of step S2c in the analysis method according to an embodiment of the present disclosure;
FIG. 9 is a diagram illustrating an example of step S2c in the analysis method according to an embodiment of the present disclosure;
FIG. 10 is a diagram illustrating an example of step S2d in the analysis method according to an embodiment of the present disclosure;
FIG. 11 is a diagram illustrating an example of step S3 in the analysis method according to an embodiment of the present disclosure;
FIG. 12 is a diagram illustrating an example of step S3 in the analysis method according to an embodiment of the present disclosure;
FIG. 13 is a diagram illustrating an example of step S3 in the analysis method according to an embodiment of the present disclosure;
FIG. 14 is a diagram illustrating an example of step S4 in the analysis method according to an embodiment of the present disclosure; and
FIG. 15 is a diagram illustrating an example of step S4 in the analysis method according to an embodiment of the present disclosure.

### EMBODIMENTS OF THE DISCLOSURE

An analysis method for a polymer composite material according to the present embodiment (which hereinafter may be referred to as "the present analysis method") is a method for analyzing a polymer composite material in which a filler is dispersed in a matrix polymer, wherein a computer executes the following steps S1 to S4.
[Step S1] a step of creating an analytical model of a polymer composite material in which a filler is dispersed in a matrix polymer.
[Step S2] a step of calculating a feature representing a dispersion form of filler particle models in the analytical model.
[Step S3] a step of performing a property simulation to calculate a physical quantity related to a mechanical property or an electrical property of the analytical model.
[Step S4] a step of creating a dataset including the physical quantity obtained from the property simulation and the feature representing a dispersion form of filler particle models.

Embodiments of the present disclosure will be described in detail below.

As used herein "X and/or Y (X and Y are any given configurations)" means at least one of X and Y, and may have three meanings: only X; only Y; and X and Y.

The present analysis method is executed by a computer. The computer is similar to those used in conventional computer-aided engineering (CAE). The present analysis method is usually executed by software and hardware working together to perform process routines stored in advance in an information processing device, such as a personal computer including a central processing unit (CPU), a ROM, a working memory, a storage device such as a magnetic disk, an input device such as a keyboard and a mouse, and a display device such as a display.

In the present analysis method, the composite material to be modeled and analyzed is a composite material in which a filler is dispersed in a polymer.

Examples of the polymer include rubbers, resins, and elastomers, specifically, but not limited to, ethylene-propylene-diene monomer ternary copolymer (EPDM), acrylic rubber, urethane rubber, styrene-butadiene-styrene block polymer (SBS), styrene-isobutylene-styrene block polymer (SIBS), styrene-butadiene (SB) copolymer, styrene-isoprene (SI) copolymer, styrene-isoprene-styrene (SIS) copolymer, styrene-ethylene-butylene (SEB) copolymer, styrene-ethylene-butylene-styrene (SEBS) copolymer, styrene-ethylene-propylene (SEP) copolymer, styrene-ethylene-propylene-styrene (SEPS) copolymer, hydrogenated copolymers of the above copolymers, ethylene-propylene copolymer (EPR), butadiene rubber (BR), isoprene rubber (IR), styrene-butadiene rubber (SBR), liquid isoprene rubber (liquid IR), liquid butadiene rubber (liquid BR), liquid styrene-butadiene rubber (liquid SBR), liquid styrene-isoprene rubber (liquid SI), liquid styrene-ethylene-propylene rubber (liquid SEP), and liquid isoprene-butadiene rubber (liquid IR-BR).

Examples of the filler include carbon black, silica, talc, calcium carbonate, carbon fiber, and carbon nanotubes. Conductive fillers include conductive carbon fillers such as conductive carbon black, carbon nanotubes, and graphite.

Examples of the composite material include materials for vibration isolation rubber used for vibration isolation applications in automobiles and the like, materials for various tubes and hoses for automobiles, materials for automobile tires, and materials for various sealant materials.

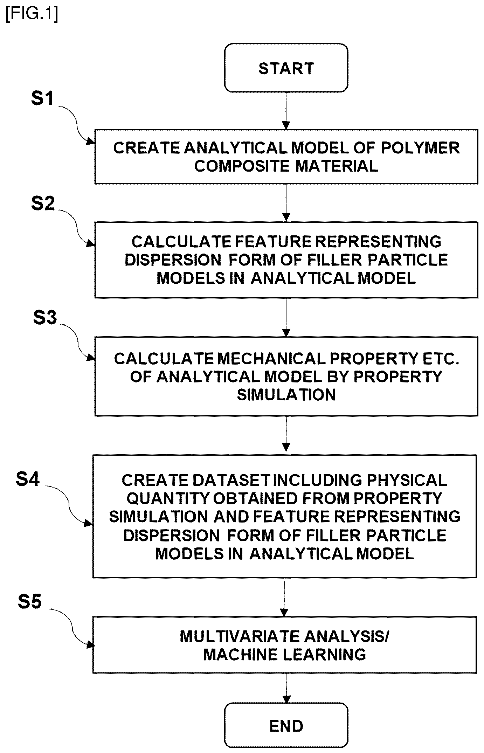

FIG. 1 is a flowchart illustrating an example of a main processing procedure of the present analysis method. The flowchart illustrates an exemplary embodiment. The present analysis method is not limited to the order in the flowchart and the like.

### <Step S1>

The present analysis method includes step S1 of creating an analytical model of a polymer composite material in which a filler is dispersed in a matrix polymer.

The analytical model according to the present analysis method is an analytical model composed of a plurality of unit elements that may be analyzed numerically by a computer in a virtual model creation region, similar to a conventional analytical model, and is created according to a conventionally known method.

The model creation region is the entire region where a periodic boundary condition is defined, or a partial region having an arbitrary shape extracted from the region where the periodic boundary condition is defined, or a partial region having an arbitrary shape extracted from a space where the periodic boundary condition is not defined. The range of the model creation region is preset.

It is preferable that the analytical model includes filler particle models and a matrix polymer model and further includes an interfacial phase model that constitutes the interface between the filler particle models and the matrix polymer model.

The filler particle models are, for example, primary particle models of the filler. The shape of the filler particle models is generated by approximately modeling a shape such as sphere, oblate sphere (long ellipsoid, oblate ellipsoid), plate-like shape, or cylindrical shape. Parameters are preset to specify a size (size of the region occupied in the model creation region) such as radius, diameter, and volume.

The filler particle models may be models of secondary particles, which are aggregates of the filler. For example, the feature related to the number of filler particle models may be the number of primary particles or the number of secondary particles.

For example, the other region excluding the regions where the filler particle models are created may set as the matrix polymer model. The interface between the filler particle models and the matrix polymer model may be set as the interfacial phase model.

As an example of the interfacial phase model, for example, among the position elements of filler particle models, a position element with at least one adjacent element being the matrix polymer model may be set as the interfacial phase model.

In addition, in execution of various property simulations described below, necessary property parameters and the like are set separately. Examples of the property parameters include elastic modulus and conductivity of each model.

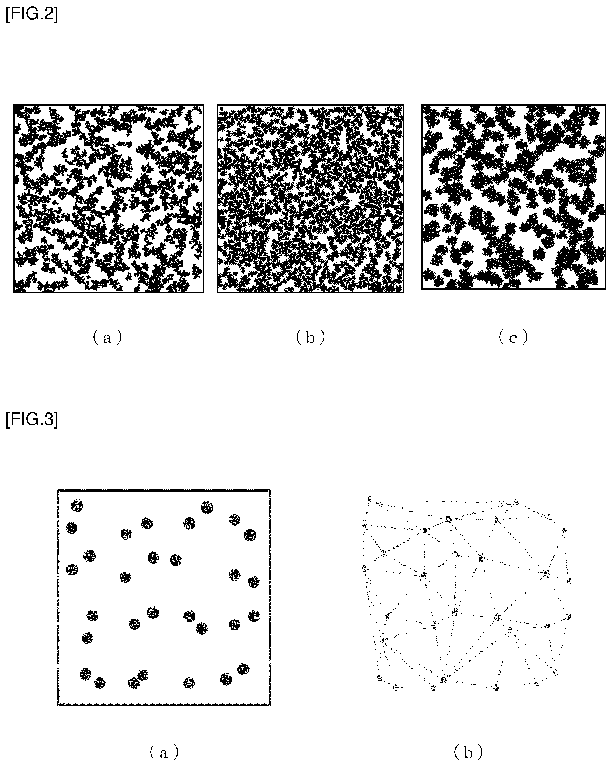

FIG. 2 is a diagram schematically illustrating an example of the analytical model. For convenience, FIG. 2 shows a two-dimensional representation of a three-dimensional (for example, rectangular parallelepipedic) model creation region and three-dimensional (for example, spherical) filler particle models (this is applicable to the figures subsequent to FIG. 2). The analytical model is created, for example, by arranging a plurality of filler particle models at random at arbitrary coordinates (X, Y, Z), based on a predetermined algorithm and parameters. In accordance with a predetermined algorithm and parameters, analytical models with different dispersion forms of filler particle models are created as shown in FIG. 2(a) to (c). For example, a plurality of analytical models with different dispersion forms of filler particle models may be created by setting the feature related to the particle size of the filler particle model and the feature related to the number of filler particle models to different numerical values as appropriate.

### <Step S2>

The present analysis method includes step S2 of calculating a feature representing a dispersion form of filler particle models in the analytical model created in step S1.

### (Step S2a: Delaunay partitioning)

An exemplary embodiment of step S2 is step S2a of setting vertices based on the coordinates of filler particle models in the analytical model and performing Delaunay partitioning. For example, Delaunay partitioning may be performed by setting vertices based on the centroid coordinates of filler particle models, which are primary particle models of the filler.

An example of step S2a is, for example, a step of performing Delaunay triangulation for the coordinates of filler particle models in a two-dimensional analytical model image (two-dimensional image) and calculating the length of a Delaunay edge and/or the area of a Delaunay triangle.

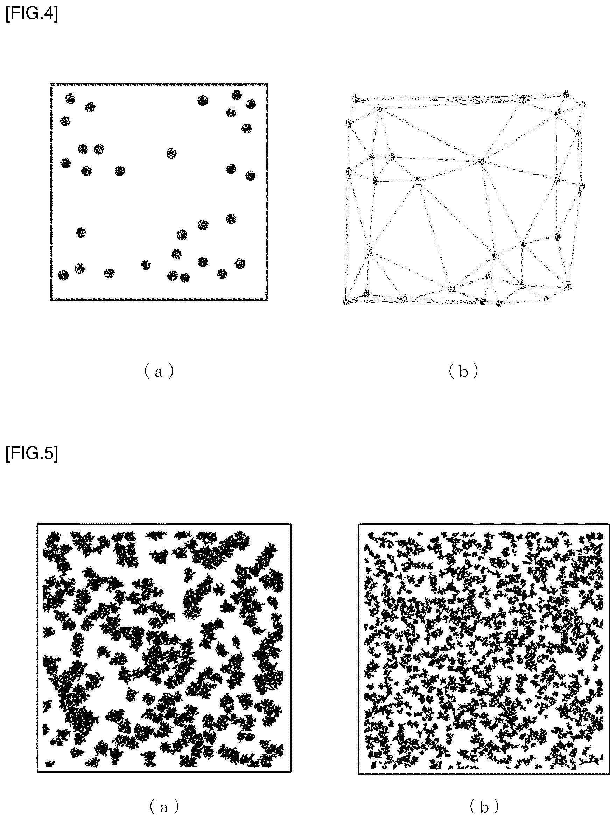

An example of step S2a will be described using a schematic diagram. For example, for image data of an analytical model having a dispersion form shown in FIG. 3(a), the centroid coordinates of filler particle models (for example, primary particle models) in the analytical model are set as vertices, and Delaunay triangulation is performed. By performing Delaunay triangulation, a plurality of Delaunay triangles shown in FIG. 3(b) are generated. Similarly, for example, for an analytical model having a dispersion form shown in FIG. 4(a), the centroid coordinates of filler particle models (for example, primary particle models) in the analytical model are set as vertices, and Delaunay triangulation is performed. By performing Delaunay triangulation, a plurality of Delaunay triangles shown in FIG. 4(b) are generated. Delaunay triangulation is executed by any geometric algorithm that connects points in space and divides the space into a group of triangles. For example, the Python library "OpenCV" may be preferably used.

Then, an average and/or a variance of the lengths (lengths of line segments between vertices) of Delaunay edges of the Delaunay triangles generated by Delaunay triangulation is calculated. Alternatively, an average and/or a variance of the areas of the Delaunay triangles generated by Delaunay triangulation is calculated.

In step S2a, the primary particles of the filler may be used as filler particle models, and Delaunay partitioning may be performed by setting arbitrary coordinates such as the centroid coordinates of the filler particle models as vertices. Alternatively, the secondary particles, which are aggregates of the filler, may be used as filler particle models, and Delaunay partitioning may be performed by setting arbitrary coordinates such as the centroid coordinates of the secondary particles as vertices.

Delaunay triangulation is executed for each of analytical model examples (two-dimensional analytical model images) shown in FIG. 5(a) and FIG. 5(b), and an average and a variance of the lengths of Delaunay edges are calculated. The results are listed in Table 1 below.

**Table 1**

| | Analytical model of FIG. 5(a) | Analytical model of FIG. 5(b) |
|---|---|---|
| Average of Delaunay edge lengths | 68.3 | 41.8 |
| Variance of Delaunay edge lengths | 37.8 | 29.0 |

**In** addition, as a result of calculating and examining the correlation of the average and the variance of the lengths of Delaunay edges with the results (physical quantity indicating the degree of stress concentration) obtained from a property simulation (simulation to predict the degree of stress concentration) to calculate a physical quantity related to a mechanical property using the analytical model, it was found that the smaller the average and the variance of the lengths of Delaunay edges, the better the durability tends to be (the less the stress concentration).

Therefore, the average and the variance of the lengths of Delaunay edges may be effective as features representing the dispersion form of filler particle models, thereby facilitating the accumulation of knowledge on the dispersion forms of fillers and contributing to the establishment of design guidelines for polymer composite materials. In addition, for example, analysis using a dataset including features related to the average and the variance of the lengths of Delaunay edges may be performed to improve the prediction accuracy of a predictive model.

The Delaunay partitioning in step S2a may be two-dimensional Delaunay triangulation for a two-dimensional analytical model or may be polyhedral partitioning such as three-dimensional Delaunay tetrahedralization for a three-dimensional analytical model.

In other words, the above example involves Delaunay partitioning in which a two-dimensional plane is divided by triangles whose vertices are coordinates discretely distributed on a two-dimensional plane. However, the Delaunay partitioning may be extended to a spatial partitioning technique for a cloud of points in three-dimensional space. In the three-dimensional extended Delaunay partitioning, three-dimensional space is partitioned by simplexes whose vertices are coordinates distributed discretely on three-dimensional space. An example of the simplex in three-dimensional space is a polyhedron such as tetrahedron. Delaunay partitioning in three-dimensional space divides three-dimensional space, for example, by tetrahedrons whose vertices are coordinates distributed discretely on three-dimensional space. Therefore, for example, in Delaunay tetrahedralization, an average and/or a variance of the lengths of edges (lengths of line segments between vertices) of tetrahedrons is calculated, or an average and/or a variance of the volumes of tetrahedrons is calculated.

### (Step S2b: Iδ index)

Another exemplary embodiment of step S2 is, for example, step S2b of calculating an Iδ index represented by formula (1). ${I}_{δ} = q {\sum }_{j = 1}^{q} {x}_{j} \left({x}_{j}-1\right) / {\sum }_{j = 1}^{q} {x}_{j} \left({\sum }_{j = 1}^{q} {x}_{j} - 1\right)$ (In formula (1), q is the number of regions of the analytical model divided into a predetermined number of regions, and Xⱼ (where j is a natural number) is the number of filler particle models in the jth region.)

The Iδ index is a value represented by formula (1), where q is the number of regions (the number of quadrats), and xⱼ (where j is a natural number) is the number of filler particle models in the jth region (quadrat). When the Iδ index is greater than 1, the distribution pattern of filler particle models is considered to be a clumped distribution. When Iδ index is 1, the distribution pattern of filler particle models is considered to be a Poisson's distribution. When Iδ index is less than 1, the distribution pattern of filler particle models is considered to be a uniform distribution.

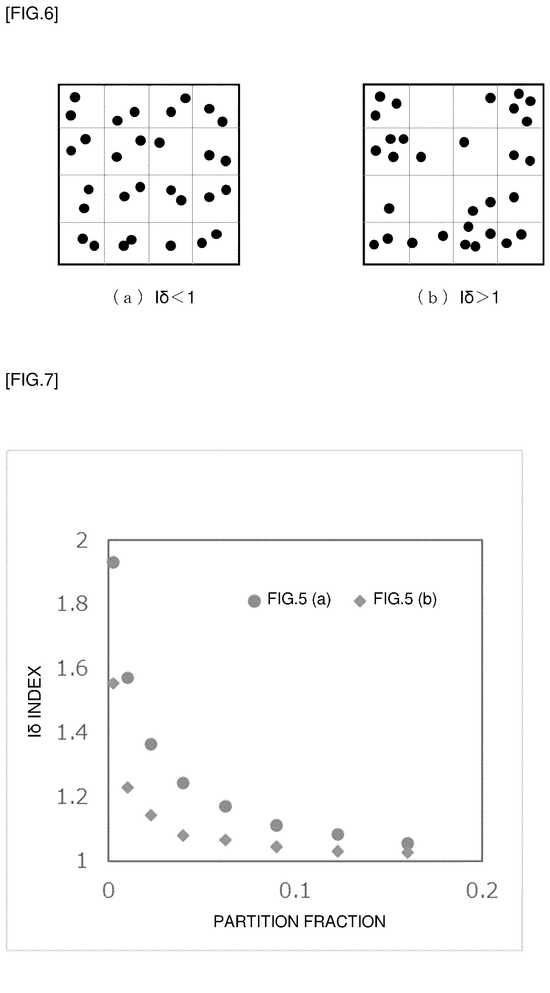

FIG. 6 is a schematic diagram illustrating an example of step S2b. FIG. 6 shows a schematic image of a two-dimensional analytical model. In step S2b, the image is divided equally into predetermined square regions (quadrats), and the number of filler particle models having centroid coordinates in each region (quadrat) is counted.

For example, referring to FIG. 6(a), the number of divided regions (number of quadrats) q is 16, the number of filler particle models in the region (quadrat) in the first row and first column in FIG. 6(a) counts 2, the number of filler particle models in the region (quadrat) in the first row and second row counts 2, and so on. Thus, the value of Iδ index represented by formula (1) is calculated.

FIG. 7 shows the results of calculating the Iδ index in step S2b for the two-dimensional analytical models shown in FIG. 5(a) and FIG. 5(b) described above.

In addition, as a result of calculating and examining the correlation of the Iδ index with the results (physical quantity indicating the degree of stress concentration) obtained from a property simulation (simulation to predict the degree of stress concentration) to calculate a physical quantity related to a mechanical property using the analytical model, it was found that the smaller the Iδ index, the better the durability tends to be (the less the stress concentration).

Therefore, the Iδ index may be effective as a feature representing the dispersion form of filler particle models, thereby facilitating the accumulation of knowledge on the dispersion forms of fillers and contributing to the establishment of design guidelines for polymer composite materials. In addition, for example, analysis using a dataset including a feature related to the Iδ index may be performed to improve the prediction accuracy of a predictive model.

The Iδ index in step S2b may be calculated for any two-dimensional analytical model as the analytical model. Alternatively, the Iδ index may be calculated, for example, based on the coordinates distributed discretely on three-dimensional space of the analytical model (three-dimensional). In this case, the region (quadrat) is, for example, a cube, and the number of filler particle models having coordinates in the cube is counted to calculate the Iδ index represented by formula (1).

In step S2b, the primary particles of the filler may be used as filler particle models, and a value of the Iδ index may be calculated based on arbitrary coordinates such as the centroid coordinates of the filler particle models. Alternatively, the secondary particles, which are aggregates of the filler, may be used as filler particle models, and a value of the Iδ index may be calculated based on arbitrary coordinates such as the centroid coordinates of the secondary particles.

### (Step S2c: Power spectrum obtained by Fourier transform)

Another exemplary embodiment of step S2 is, for example, step S2c of calculating at least one of a radial component and an angular component of a power spectrum obtained by a Fourier transform.

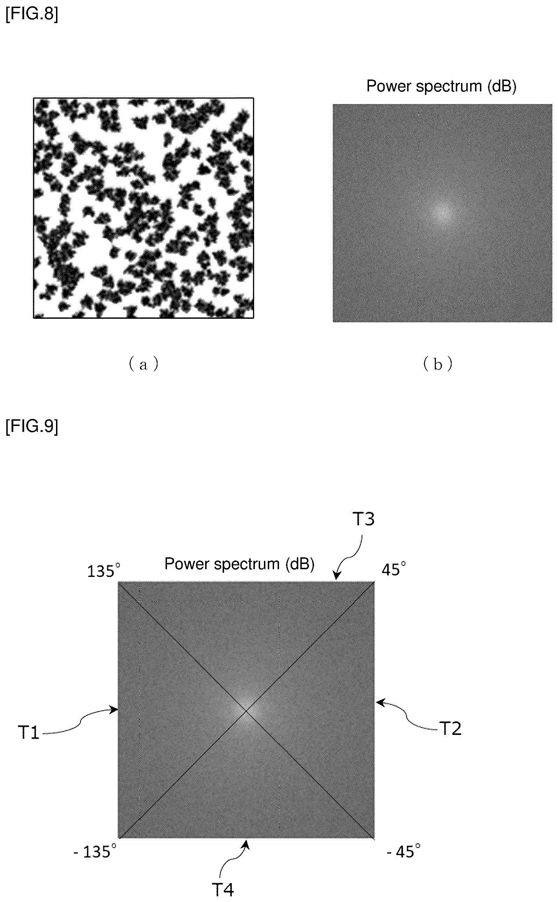

FIG. 8 is a diagram illustrating an example of step S2c. A two-dimensional Fourier transform is performed based on image data of an analytical model having a dispersion form shown in FIG. 8(a). By performing the two-dimensional Fourier transform, a power spectrum shown in FIG. 8(b) is generated. FIG. 8(b) shows a power spectral image in which low-frequency components are distributed near the center and high-frequency components are distributed outward. The Fourier transform uses a well-known method such as a fast Fourier transform (FFT).

Next, a feature representing the dispersion form is calculated based on the distribution of the power spectrum. For example, a normal method may be used to extract at least one of a radial distribution and an angular distribution of the two-dimensional power spectrum in the power spectral image generated based on the analytical model.

For example, from the viewpoint of calculating a feature related to anisotropy of the analytical model, in a step of extracting angular components in a predetermined angular range, integrating power spectra for each angle, and calculating an average of the power spectra in the range, it is possible to use a step of extracting and integrating angular components in a predetermined angular range and calculating an average of power spectra in the predetermined angular range, and, in addition, extracting and integrating angular components in another predetermined angular range different from the predetermined angular range, calculating an average of power spectra in the range, and calculating the ratio between these averages.

Specifically, for example, referring to FIG. 9, in a 400 pixel × 400 pixel power spectral image generated based on an analytical model, first, angular components in the angular range from -45° to (+)45° and angular components in the angular range from (+)135° to - 135° with respect to the horizontal axis (0° on the right of the horizontal axis) may be extracted and integrated to calculate an average (PA1) of the power spectra for both ranges. Next, angular components in the angular range from (+)45° to (+)135° and angular components in the angular range from -45° to -135° may be extracted and integrated to calculate an average (PA2) of the power spectra for both ranges. Then, the ratio between both averages (PA1/PA2) may be calculated to calculate a feature related to anisotropy, which is the ratio between longitudinal and lateral components.

In other words, referring to FIG. 9, among four triangular regions T1 to T4 separated by diagonal lines intersecting at the center point of the power spectral image, power spectra of angular components in regions T1 and T2 are extracted and integrated, and their average (PA1) is calculated; power spectra of angular components in regions T3 and T4 are extracted and integrated, and their average (PA2) is calculated; and the ratio between the averages (PA1/PA2) is calculated. The larger the ratio between the averages (PA1/PA2), the stronger the anisotropy of longitudinal components.

As a result of calculating and examining the correlation of the ratio between the averages with the results (physical quantity indicating the degree of stress concentration) obtained from a property simulation (simulation to predict the degree of stress concentration) to calculate a physical quantity related to a mechanical property using an analytical model, it was found that the weaker the longitudinal components, the better the durability tends to be (the less the stress concentration in relation to longitudinal extension change).

Therefore, the power spectrum may be effective as a feature representing the dispersion form of filler particle models, thereby facilitating the accumulation of knowledge on the dispersion forms of fillers and contributing to the establishment of design guidelines for polymer composite materials. In addition, analysis using a dataset including a feature related to the power spectrum may be used to improve the prediction accuracy of a predictive model.

For example, referring to FIG. 9, in the power spectral image generated based on an analytical model, step S2c may be a step of extracting and integrating power spectra in a predetermined angular range and power spectra in a predetermined radial range with respect to the horizontal axis (0° on the right of the horizontal axis). Specifically, for example, in a 400 pixel × 400 pixel power spectral image generated based on an analytical model, step S2c may be a step of extracting and integrating power spectra in the angular range from - 15° to 15° with respect to the horizontal axis (0° on the right of the horizontal axis) and power spectra in the radial range from 0 pixels to 30 pixels.

As a result of calculating and examining the correlation of the power spectra with the results (physical quantity indicating the degree of stress concentration) obtained from a property simulation (a simulation to predict the degree of stress concentration) to calculate a physical quantity related to a mechanical property using an analytical model, it was found that the higher the correlation with the power spectra in the angular range from -15° to 15° with respect to the horizontal axis (0° on the right of the horizontal axis) and the power spectra in the radial range from 0 pixels to 30 pixels, and the less the longitudinal periodic structure, the better the durability tends to be (in relation to longitudinal extension change).

Therefore, the power spectrum may be effective as a feature representing the dispersion form of filler particle models, thereby facilitating the accumulation of knowledge on the dispersion forms of fillers and contributing to the establishment of design guidelines for polymer composite materials. In addition, analysis using a dataset including a feature related to the power spectrum may be used to improve the prediction accuracy of a predictive model.

Step S2c may be performed for any two-dimensional analytical model as the analytical model or may be performed, for example, for an analytical model (three-dimensional). In other words, step S2c may be performed for data of a two-dimensional array structure with coordinates in x- and y-directions of the analytical model as parameters or may be performed for data of a three-dimensional array structure with coordinates in x-, y-, and z-directions as parameters.

### (Other embodiments of step S2)

Another exemplary embodiment of step S2 is, for example, step S2d of calculating a feature based on persistent homology analysis.

For example, persistent homology analysis (PH) may be performed for image data corresponding to an analytical model, and information including Birth time, Death time, and Birth-Death pair may be calculated as features.

In short, persistent homology analysis (PH) may quantify the connection of figures present in an analytical model image. PH focuses on a "hole" created as the figures are gradually grown. When the figures are grown, the figures are connected to each other to create a hole (birth). As the figures are further grown, the hole disappears (death). For a hole that originally exists, as the figures are diminished, the hole is disconnected at some timing. In other words, it is understood that the hole is created (birth) immediately after the timing. The timing of being created is recorded as "Birth Time," and the timing of disappearing is recorded as "Death Time." These may be used as features.

In this step S2d, for example, a binary image corresponding to an analytical model is used. Specifically, a black part in the image is a filler particle model, and a white part is a matrix polymer model. When the filler particle model is focused on, the black part is grown or diminished so that a feature is extracted. When the matrix polymer model is focused on, the white part is grown or diminished so that a feature is extracted.

More specifically, for example, persistent homology analysis (PH) may be performed using an image subjected to binarization as input information. Data are plotted on a plane to generate a diagram with Birth Time on the horizontal axis and Death Time on the vertical axis (persistent diagram). Then, the persistent diagram is vectorized to extract a feature.

The binarization is not limited to a particular method. For example, known methods such as adaptive binarization and Otsu's binarization may be used. Among these, adaptive binarization is preferable. Adaptive binarization sets local regions of an arbitrary size in the entire image, calculates a threshold for each local region, and binarizes each image. The threshold may be determined by calculating an arithmetic mean, or a weighted mean (Gaussian distribution) of the local region may be used as the threshold. The threshold may be set as appropriate.

In addition, it is preferable to perform noise reduction processing in a preceding process of the binarization. A known method may be used in the noise reduction processing, and the method is not particularly limited. Specifically, noise-cutting filters may be used for filtering, and examples include non-local-mean filter, average filter, Gaussian filter, median filter, band-pass filter, open filter, close filter, and bilateral filter.

The noise reduction processing and the binarization may be executed, for example, using the Python library "OpenCV." For example, for image data corresponding to an analytical model (size: 4008 pixels × 2672 pixels), non-local mean denoising may be executed with setting conditions of filter strength parameter h=10, template window size: 11, and search window size: 21, and then adaptive binarization may be executed with setting conditions of block size: 131, constant C (threshold correction): 5, and threshold calculation: arithmetic mean.

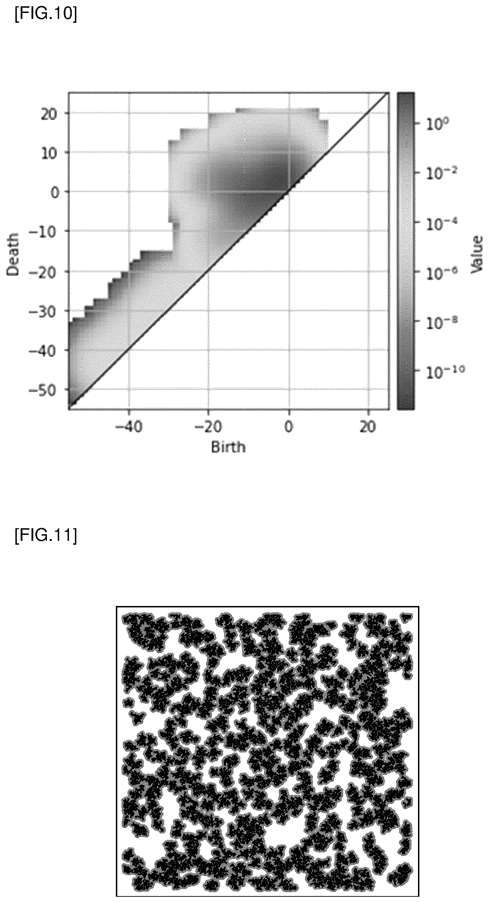

The persistent homology analysis based on the binarized image may be executed using the Python library "HomCloud." For example, a persistent diagram may be created using the binary image obtained above as input. FIG. 10 shows an example of the persistent diagram created using "HomCloud," focusing on filler particle models. The argument of distance#transform() is signed=True.

The persistent diagram may be vectorized, for example, using the following formulas. $ρ \left(x, y\right) = {\sum }_{k = 1}^{l} w \left({b}_{k}, {d}_{k}\right) exp \left(-\frac{{\left({b}_{k}-x\right)}^{2} + {\left({d}_{k}-y\right)}^{2}}{2 {σ}^{2}}\right) ,$ $w \left(b, d\right) = arctan \left(C{\left(d-b\right)}^{p}\right) .$

For example, vectorization is performed using **PI** (Persistence Image) in "HomCloud" (see, for example, Henry Adams et al. "Persistence images: a stable vector representation of persistent homology" in J. Mach Learn Res. 18 (2017), Paper No. 8, 35). The conditions for the vectorization include, for example, x#range=(-50, 20) and xbins=70. In addition, the parameters represented by σ, C, and p in the above formulas are, for example, σ=3.0, C=0.001, and p=4. The values of x#range and xbins are the conditions for how to divide the persistent diagram and, if necessary, modified as appropriate with reference to maximum and minimum values of all persistent diagrams to be analyzed.

Persistent homology is one of topological data analyses and described in detail in various literature (for example, "Persistent Homology and Its Applications to Materials Science" (Bulletin of the Japan Institute of Metals), Vol. 58, No. 1, 2019, and "Statistical Machine Learning on Persistent Diagram," Chuo University, The Institute of Statistical Mathematics, Internet <URL: https://www.math.chuo-u.ac.jp/ENCwMATH/EwM70#Fukumizu.pdf>).

The persistent homology analysis in step S2d may be performed for any two-dimensional analytical model as the analytical model or may be performed, for example, for an analytical model (three-dimensional). In other words, the persistent homology process may be applied to data of a two-dimensional array structure with coordinates in x- and y-directions of the analytical model as parameters, or the persistent homology process may be applied to data of a three-dimensional array structure with coordinates in x-, y-, and z-directions as parameters.

In step S2, it is preferable that at least one selected from the group consisting of steps S2a, S2b, S2c, and S2d is used.

In step S2, at least two or more or three or more selected from the group consisting of steps S2a, S2b, S2c, and S2d may be used.

### <Step S3>

The present analysis method includes step S3 of performing a property simulation to calculate a physical quantity related to a mechanical property such as durability and viscoelasticity or an electrical property such as conductivity of the analytical model.

The property simulation may use a conventionally known method as appropriate. For example, a finite element method (FEM) is used to divide the analytical model into a predetermined mesh, and the filler particle models, the matrix polymer model, and the values of material constants and material parameters of the interfacial phase model, as well as tensile conditions, voltage application conditions, and the like are set to calculate various properties.

More specifically, examples include property simulations using phase field methods described in JP-B-7382478 and JP-B-7197871.

### <Step S4>

The analysis method according to the present embodiment includes step S4 of creating a dataset including, for example, the physical quantity obtained from the property simulation as an objective variable and the feature representing a dispersion form of filler particle models in the analytical model as an explanatory variable. For example, datasets shown in Table 2 are created for a plurality of analytical models (A, B, C ...) with different dispersion forms.

**Table 2**

| Analytical model | Delaunay edge length | Iδ index | Power spectrum | Particle size of filter particle models | ... | Mechanical property simulation |
|---|---|---|---|---|---|---|
| A | data a1 | data a2 | data a3 | data a4 | ... | data a5 |
| B | data b1 | data b2 | data b3 | data b4 | ... | data b5 |
| C | data c1 | data c2 | data c3 | data c4 | ... | data c5 |
| . | . | . | . | . | . | . |
| . | . | . | . | . | . | . |
| . | . | . | . | . | . | . |

It is preferable that step S4 is a step of creating a dataset including the physical quantity obtained from the property simulation, the feature representing a dispersion form of filler particle models (at least one of S2a to S2d), a feature related to the particle size of filler particle models, a feature related to the number of filler particle models, and a feature related to the thickness of the interfacial phase model. The dataset may include a feature other than those listed above. For example, the dataset may include a feature related to a filler filling rate (for example, a volume filling rate of filler particle models in the analytical model).

### <Step S5>

It is preferable that the present analysis method includes step S5a of performing multivariate analysis based on the dataset. The multivariate analysis may use various statistical analysis methods as appropriate, such as multiple regression analysis, canonical correlation analysis, logistic regression analysis, and quantification theory type I.

It is preferable that step S5a is a step of creating a predictive model by performing multivariate analysis using a dataset including the physical quantity related to a mechanical property or an electrical property obtained from the property simulation as an objective variable, and the feature related to a dispersion form and the feature related to the number of filler particle models as explanatory variables.

The present analysis method may include step S5b of performing machine learning based on the dataset. The machine learning may use various methods as appropriate, such as neural networks.

It is preferable that step S5b is a step of creating a predictive model by performing machine learning using a dataset including the physical quantity related to a mechanical property or an electrical property obtained from the property simulation, the feature representing a dispersion form, and the feature related to the number of filler particle models.

### EXAMPLES

### [Example 1]

A plurality of analytical models were created based on the method according to the present embodiment. Specifically, a total of 405 analytical models were created, in which the particle size (primary particle size) of filler particle models had multiple levels (3 levels: 10 nm, 15 nm, 30 nm), the number of dispersed particles (the number of aggregates in the structure, corresponding to "the number of filler particle models") had multiple levels (3 levels: 200, 600, 1000), the thickness of the interfacial phase model had multiple levels (3 levels: 5 nm, 10 nm, 15 nm), the elastic modulus of the interfacial phase model had multiple levels (3 levels: 1.5 MPa, 1.5 MPa, 2.5 MPa), and the number of samples (N) was 5. In each analytical model, the elastic modulus of the filler particle models was set to 8 MPa.

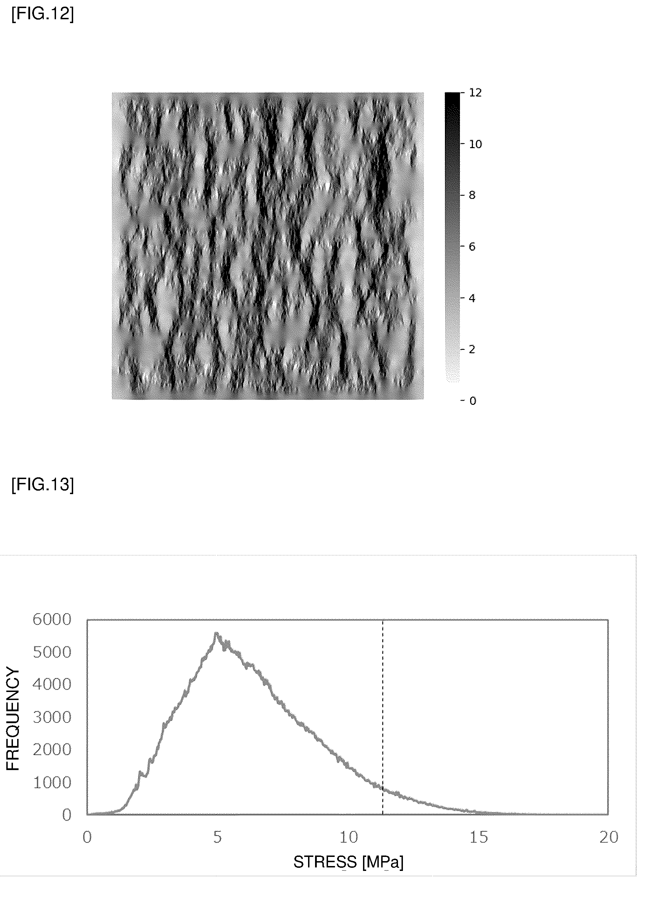

Next, a property simulation was performed to calculate a physical quantity related to a mechanical property of the analytical model. Specifically, using phase-field analysis application software, the analytical model was tensile deformed in the vertical direction (constant strain (strain 1.5)), and stress simulation calculations were performed to obtain stress distribution information of the analytical model. FIG. 11 shows an exemplary analytical model, and FIG. 12 shows the stress distribution information obtained from the analytical model.

A histogram was generated based on the obtained stress distribution information (see FIG.13), and the top 5% stress (right region of the dashed line in FIG.13) was divided by a mean stress. The value obtained as described above is a value indicating the degree of stress concentration, and the smaller the value, the more the stress is distributed without concentration, and the better the durability.

On the other hand, based on the method according to the foregoing embodiment, an average and a variance of lengths of Delaunay edges, the Iδ index, and the angular components of power spectra (T1 and T2) were calculated for each analytical model.

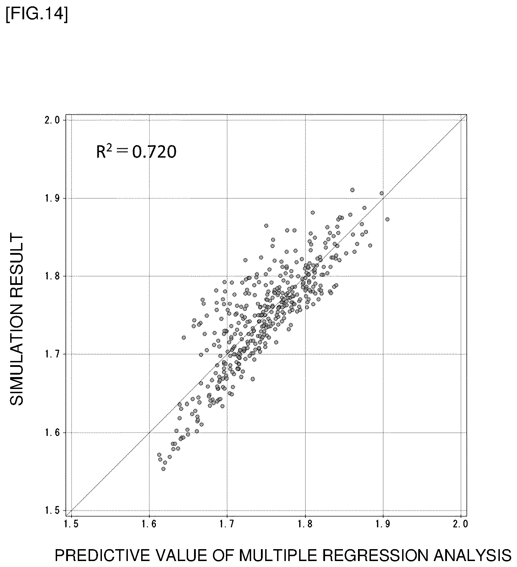

Next, multiple regression analysis was performed. In other words, the particle size of filler particle models, the number of dispersed particles, the thickness of the interfacial phase model, the elastic modulus of the interfacial phase model, the average and the variance of lengths of Delaunay edges, the Iδ index, and the angular components of power spectra (averages in regions T1 and T2 described above) were set as explanatory variables. The multiple regression analysis was performed by selecting the number of dispersed particles, the thickness of the interfacial phase model, the elastic modulus of the interfacial phase model, the average of lengths of Delaunay edges, the Iδ index, and the angular components of power spectra (averages in regions T1 and T2 described above) based on the forward-backward stepwise method, and setting the value indicating the degree of stress concentration obtained by stress simulation calculations as an objective variable. The results are listed in FIG. 14.

### [Comparative Example 1]

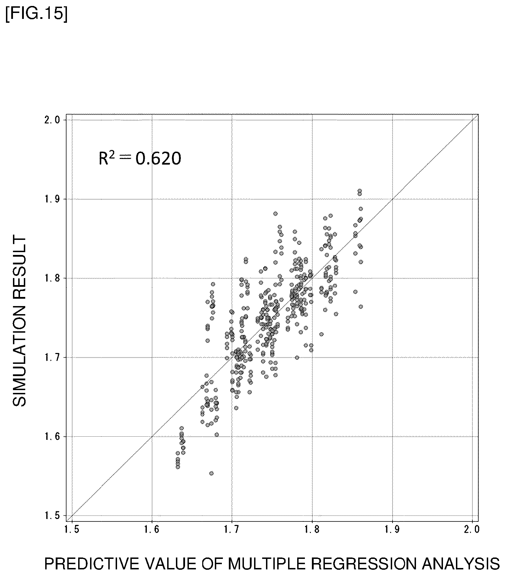

On the other hand, in Comparative Example 1, multiple regression analysis was performed without using the feature representing a dispersion form of filler particle models. In other words, multiple regression analysis is performed by changing the explanatory variables in Example 1. Specifically, in Comparative Example 1, multiple regression analysis was performed without using the average and the variance of lengths of Delaunay edges, the Iδ index, and the angular components of power spectra (T1 and T2) as explanatory variables. The results are listed in FIG. 15.

FIG. 14 and FIG. 15 show that the coefficient of determination is improved by using the lengths of Delaunay edges, the Iδ index, and the angular components of power spectra (T1 and T2) as explanatory variables. This proves the usefulness of the analysis including the features representing the dispersion form.

In the above example, specific forms in the present disclosure are shown. The above example is merely illustrative and is not to be construed as limiting. Various modifications apparent to those skilled in the art are contemplated to be within the scope of the present disclosure.

### INDUSTRIAL APPLICABILITY

According to an embodiment of the present disclosure, a new method for analyzing the dispersion form of a filler in a polymer composite material in which the filler is dispersed in a matrix polymer is provided. This method is useful because it facilitates the accumulation of knowledge on the dispersion forms of fillers and contributes to the establishment of design guidelines for polymer composite materials. According to an embodiment of the present disclosure, this method is also useful in that the prediction accuracy of a predictive model may be improved.

## Claims

1. A computer-implemented method for analyzing a polymer composite material in which a filler is dispersed in a matrix polymer, the method comprising the steps of:
Creating an analytical model of a polymer composite material in which a filler is dispersed in a matrix polymer;
Calculating a feature representing a dispersion form of filler particle models in the analytical model;
Performing a property simulation to calculate a physical quantity related to a mechanical property or an electrical property of the analytical model; and
Creating a dataset including the physical quantity related to a mechanical property or an electrical property obtained from the property simulation and the feature representing a dispersion form of filler particle models.

2. The method for analyzing a polymer composite material according to claim 1,
wherein the analytical model is an analytical model comprising a matrix polymer model and filler particle models, and
wherein the step of creating a dataset includes creating a dataset comprising the physical quantity related to a mechanical property or an electrical property obtained from the property simulation,
the feature representing a dispersion form of filler particle models, and a feature related to a number of filler particle models.

3. The method for analyzing a polymer composite material according to claim 2, further comprising a step of performing multivariate analysis or machine learning using the dataset to create a predictive model.

4. The method for analyzing a polymer composite material according to claim 3, wherein the step of calculating a feature representing a dispersion form of filler particle models in the analytical model comprises setting vertices based on coordinates of the filler particle models and calculating a feature based on Delaunay partitioning.

5. The method for analyzing a polymer composite material according to claim 3,
wherein the step of calculating a feature representing a dispersion form of filler particle models in the analytical model comprises calculating a feature based on an Iδ index represented by formula (1): ${I}_{δ} = q {\sum }_{j = 1}^{q} {x}_{j} \left({x}_{j}-1\right) / {\sum }_{j = 1}^{q} {x}_{j} \left({\sum }_{j = 1}^{q} {x}_{j} - 1\right)$
wherein in formula (1), q is a number of regions of the analytical model divided into a predetermined number of regions, and Xⱼ is a number of filler particle models in a jth region.

6. The method for analyzing a polymer composite material according to claim 3,
wherein the step of calculating a feature representing a dispersion form of filler particle models in the analytical model comprises:
calculating a feature based on at least one of a radial component and an angular component of a power spectrum obtained by a Fourier transform.

7. The method for analyzing a polymer composite material according to any one of claims 4 to 6,
wherein the step of performing a property simulation to calculate a physical quantity related to a mechanical property of the analytical model includes,
deforming the analytical model, and
calculating a physical quantity related to stress of the deformed analytical model.

8. The method for analyzing a polymer composite material according to claim 7, wherein the polymer composite material in which a filler is dispersed in a matrix polymer is a vibration isolation rubber.
